# EUROPÄISCHE PATENTSCHRIFT

(11) **EP 1 394 180 B1**
(45) Veröffentlichungstag und Bekanntmachung des Hinweises auf die Patenterteilung: **16.07.2008**
(21) Anmeldenummer: 02017525.3
(22) Anmeldetag: 06.08.2002
(51) Int. Cl.: C07K 14/55, C07K 14/705, A61K 47/48, A61K 38/20, A61K 38/19

(54) **Synthetische Mimetika von physiologischen Bindungsmolekülen**
Synthetic mimetic molecules of physiological binding molecules
Molécules synthétiques mimetiques de molécules de liason physiologiques

(43) Veröffentlichungstag der Anmeldung: 03.03.2004
(73) Patentinhaber: AplaGen GmbH, 52499 Baesweiler (DE)
(72) Erfinder: Haberl, Udo, Dr. rer. nat., 52499 Baesweiler (DE); Frosch, Christian, 76187 Karlsruhe (DE); Frank, Hans-Georg, Prof. Dr. med., NL-6462 EL Kerkrade (NL)
(74) Vertreter: Meyers, Hans-Wilhelm

(56) Entgegenhaltungen:
- WO-A-00/04048
- WO-A-00/24782
- WO-A-00/39278
- WO-A-93/21206
- WO-A-98/20036
- US-A- 6 096 710
- CIARDELLI T L ET AL: "Structural studies of interleukin 2" PEPTIDES. STRUCTURE & FUNCTION, PROCEEDINGS OF THE AMERICAN PEPTIDE SYMPOSIUM, ROCKFORD, PIERCE CHEMICAL CO, US, Bd. PROC. 9, 1985, Seiten 75-78, XP002100685
- FUJII H. ET AL: 'ANTIMETASTATIC ACTVITIES OF SYNTHETIC ARG-GLY-ASP-SER (RGDS) AND ARG-LEU-ASP-SER(RLDS) PEPTIDE ANALOGUES AND THEIR INHIBITORY MECHANISMS' BIOLOGICAL & PHARMACEUTICAL BULLETIN (OF JAPAN), PHARMACEUTICAL SOCIETY OF JAPAN Bd. 18, Nr. 12, Dezember 1995, Seiten 1681 - 1688, XP001108987

### SEQUENZPROTOKOLL

<110> AplaGen GmbH
<120> Bindungsmoleküle
<130> 021112ep ME/BM
<140> 02017525.3
   <141> 2002-06-08
<160> 5
<170> PatentIn Ver. 2.1
<210> 1
   <211> 30
   <212> PRT
   <213> Künstliche Sequenz
<220>
   <223> Beschreibung der künstlichen Sequenz: Peptid 1
<400> 1
   Ala Pro Thr Ser Ser Ser Thr Lys Lys Thr Gln Leu Gln Leu Glu His
     1               5                  10                  15
   Leu Leu Leu Lys Leu Gln Met Ile Leu Asn Gly Ile Asn Asn
                20                  25                  30
<210> 2
   <211> 30
   <212> PRT
   <213> Künstliche Sequenz
<220>
   <223> Beschreibung der künstlichen Sequenz: Peptid 2
<400> 2
   Ala Pro Thr Ser Ser Ser Thr Lys Lys Thr Gln Leu Gln Leu Glu His
    1               5                  10                  15
   Phe Leu Leu Asp Phe Gln Met Ile Leu Asn Gly Ile Asn Asn
               20                  25                  30
<210> 3
   <211> 30
   <212> PRT
   <213> Künstliche Sequenz
<220>
   <223> Beschreibung der künstlichen Sequenz: Peptid 3
<400> 3 -
<210> 4
   <211> 6
   <212> PRT
   <213> Künstliche Sequenz
<220>
   <223> Beschreibung der künstlichen Sequenz: Cyclohexapeptid
<400> 4
   Asp Lys Asp Lys Asp Lys
    1               5
<210> 5
   <211> 25
   <212> PRT
   <213> Künstliche Sequenz
<220>
   <223> Beschreibung der künstlichen Sequenz: Seitenkettenuntereinheit des TRAILR-spezifischen Bindungsmolekül
<400> 5
   Ser Lys Asn Glu Lys Ala Leu Gly Arg Lys Ile Asn Ser Trp Glu Ser
    1               5                  10                  15
   Ser Arg Ser Gly His Ser Phe Leu Ser
               20                  25

## Beschreibung

Bei zahlreichen Erkrankungen kommt es im Rahmen des pathogenetischen Geschehens zur Veränderung der Wirkspiegel von Antikörpern, Enzymen und von endo- para- oder autokrinen Botenstoffen. Solche biologisch aktiven Bindungsmoleküle können aus der Stoffklasse der Proteine (Beispiele: Insulin, Wachstumsfaktoren, Zytokine, Releasing-Hormone, Antikörper, Enzyme etc.), der Klasse der Lipide (Prostaglandine und verwandte Fettsäurederivate), der Klasse der Steroide (Beispiele: Glucocorticoide, Mineralocorticoide) stammen. Im folgenden soll der Begriff des Zytokins prototypisch für biologisch aktive Bindungsmoleküle interpretiert werden, die in Bindungsvorgänge oder Signaltransduktionsvorgänge involviert sind. Zytokine sind biologische Botenstoffe mit hoher Spezifität und definierter Wirkung. Sie erzeugen ihre biologische Wirkung durch Bindung an Zytokin-spezifische Rezeptoren, die vorzugsweise an der Oberfläche von Zielzellen präsentiert werden. Kommt es zum Kontakt des Botenstoffes mit dem Rezeptor löst dieser im typischen Fall eine intrazelluläre Signaltransduktionskaskade aus, die zum biologischen Effekt in der Zelle bzw. im Gewebe führt. Typische Effekte, die durch solche Rezeptoren ausgelöst werden können, sind zum Beispiel der Eintritt einer Zelle in den Zellzyklus und damit die Zunahme der Vermehrungsgeschwindigkeit oder aber auch die Auslösung der Apoptose, einer Form des Zelltodes. Auch im Entzündungsgeschehen, im Immunsystem und in vielen lokalen Regulationsvorgängen des Säugetierkörpers spielen Zytokine eine wichtige Rolle in der Homöostase des betreffenden Organismus. Bei vielen Zytokinen, so z.B. bei den meisten Mitgliedern der Zytokinfamilie der Interleukine, besteht der Rezeptor an der Zelloberfläche aus verschiedenen Proteinen, die durch die Bindung des Zytokins zusammengeführt werden. Viele Zytokine haben daher spezifische Bindungsstellen, die an zwei oder mehr Rezeptorproteine gleichzeitig binden müssen, um die Wirkung mit hoher Effizienz auszulösen.

Das bedeutet, daß bei der Zytokin-Rezeptor Interaktion genau definierte Entfernungen und räumliche Orientierung der verschiedenen bindenden Regionen im Zytokinmolekül zueinander für die volle Entfaltung der Wirkung kritisch und erforderlich sind. Bei einigen Zytokinen kommt hinzu, dass auch das Zytokin selbst aus verschiedenen Proteinen besteht, die im Rahmen der Rezeptorinteraktion zusammentreten müssen¹. In jüngster Zeit wurden eine Reihe von Zytokinen rekombinant hergestellt, um sie als Arzneimittel einzusetzen. Indikationen, in denen solche Arzneimittel bereits eingesetzt werden sind beispielsweise schwere lebensbedrohliche Tumorerkrankungen und Immunschwächeerkrankungen.
¹ C.Aul, W. Schneider (Eds.) Interferons, Biological Activities and Clinial Efficacies, 1997, Springer Verlag Berlin

US 6,096,710 offenbart Bindungsmoleküle, die Benzol-1,3,5-tricarbonsäure (TMA) als zyklische Einheit und 6-Aminohexansäure als Linker aufweisen.

Allerdings weist die rekombinante Herstellung eine Reihe von Nachteilen auf:
1. Die Herstellung rekombinanter Proteine - z. B. Zytokine - erfordert einen hohen Aufwand beim "set-up". Es muss auf aufwändige Weise sichergestellt werden, dass die zur Expression verwendeten Zellen genau das Protein in der exakten "nativen" Konformation herstellen. Eine Vielzahl von Tests ist nötig, um ein geeignetes Expressionssystem mit Zellen und einem Vektor, sowie Bedingungen für eine hohe Expression und Stabilität in Lösung aufzufinden. Oft sind die "set-up" Bedingungen nicht auf eine Fermentation im größeren Maßstab übertragbar. Selbst wenn optimale Bedingungen aufgefunden wurden, erweisen sich die Expressionssysteme oft als labil und störanfällig.
2. Da die Zytokine in regelmäßiger Folge verabreicht werden müssen, muß gewährleistet sein, dass sie nach der Expression, die regelmäßig in Bakterien erfolgt, aufgereinigt werden und dann absolut frei von bakteriellen Kontaminationen sind, um eine systemische Allergie zu vermeiden. Dadurch müssen komplexe Methoden eingesetzt werden, die sich enorm auf die Preisgestaltung auswirken.
3. Viele Botenstoffe, wie z. B. Zytokine, besitzen nicht nur eine Bindungsstelle für den Rezeptor, sondern es sind weitere, meistens nicht vollständig verstandene Domänen vorhanden, die möglicherweise andere Signalwege induzieren können als die, die beabsichtigt sind. Damit könnten u. a. auch die häufig auftretenden Nebenwirkungen erklärt werden, die teilweise eine Behandlung mit diesen Substanzen unmöglich machen.
4. Rekombinant hergestellte Proteine weisen häufig eine vom nativen Protein abweichende Tertiärstruktur auf und werden deswegen vom menschlichen Immunsystem als "fremd" erkannt. Die dadurch induzierten Antikörper neutralisieren das Zytokin und führen so zu einem Verlust der Wirksamkeit des Cytokins.
5. Rekombinante Proteine zeigen oft eine auffällige Labilität gegenüber proteolytischen - also eiweißabbauenden - Enzymen. Dadurch ist eine häufige Verabreichung in relativ hohen Konzentrationen notwendig, die zum einen den Patienten sehr belastet, zum anderen die Therapie sehr kostenintensiv gestaltet.

Eine preiswerte Alternative zur rekombinanten Herstellung eines Zytokins sind organisch-chemisch zugängliche vollsynthetische Mimetika biologisch aktiver Proteine wie z.B. der Zytokine.

Ein Weg, der dabei beschritten wird, ist z.B. das künstliche Design katalytisch aktiver Zentren bekannter Proteine, wobei hier die Forschung noch relativ am Anfang steht und bisher lediglich Stabilisierungen von peptidischen Strukturen (β-Sheet, Helices, Turns) und in einigen Fällen gezielte Komplexierungen kleinerer Moleküle realisiert wurden.

Ein weiterer Ansatz ist das rechnergestützte Design kleiner spezifischer Bindungsmoleküle von zum Beispiel peptidischer Natur. Solche kleinen spezifischen Bindungsmoleküle haben im allgemeinen eine Sequenz von weniger als 100 Aminosäuren. Aufgrund ihrer Größe können solche Moleküle aber nicht zur Assoziation von Rezeptoruntereinheiten beitragen, wie das für die volle Zytokinwirkung erforderlich wäre. Sie sind im Regelfalle auch nicht so zu konstruieren, dass sie für zwei oder mehr Bindungsstellen auf einem großen Rezeptorkomplex gleichzeitig hochaffin sind.

Peptide lassen sich inzwischen mit zahlreichen standardisierten Methoden recht einfach chemisch synthetisieren, obwohl für jedes einzelne Peptid eine Peptide lassen sich inzwischen mit zahlreichen standardisierten Methoden recht einfach chemisch synthetisieren, obwohl für jedes einzelne Peptid eine individuelle Synthesestrategie ausgearbeitet werden muß. In der Regel werden die Peptide dabei an Festphasen synthetisiert, wobei die Arbeitsschritte Aktivierung einer N-terminal geschützten Aminosäure, Kopplung, Waschen, Deblockieren, Aktivierung usw. so oft durchlaufen werden, bis das gewünschte Peptid fertiggestellt ist. Dieses wird von der Festphase abgelöst, über HPLC gereinigt und dann den weiteren Untersuchungen wie z. B. Verifizierung der Sequenz und biologische Tests zugeführt. Die Synthese ist im allgemeinen umso einfacher und zuverlässiger, je kürzer das Peptid ist.

In einigen wenigen Untersuchungen über Peptid-Oligomere konnte gezeigt werden, daß chemisch synthetisierte und oligomerisierte Peptide biologische Aktivität zeigten, wobei diese regelmäßig niedriger war, als die des nativen Proteins, so z. B. im Falle von Erythropoetin², jedoch oft höher als die Wirkung des Monomers.
² Patent Nr. WO 96/40772

Die Herstellung der Oligomere erfolgt bislang ebenfalls nach standardisierten, in der Literatur beschriebenen Methoden mittels zum Großteil kommerziell erhältlicher "cross-linker" (Vernetzer). Häufig sind das biologisch inerte und "unauffällige" Molekülstrukturen, die im Körper nicht vorkommen, mit der Intention, dass die spezielle chemische Zusammensetzung keine immunologischen Reaktionen hervorruft.

Peptide haben *in vivo* eine extrem kurze Halbwertszeit, d. h., sie werden sehr schnell von körpereigenen Enzymen abgebaut und ausgeschieden. Um Peptide und Proteine metabolisch zu stabilisieren, werden verschiedene Ansätze angewandt. Zum einen werden nicht natürliche Aminosäuren bei der Synthese eingesetzt, die nicht gespalten werden können, zum anderen werden Proteine mit inerten chemischen Gruppen modifiziert, um sie gegen Abbau zu schützen. Ein bekanntes Beispiel dafür ist das PEG-Intron (Schering-Plough), ein Interferon, das mit Polyethylenglykol modifiziert wurde.

Ansätze, dieses Problem synthetisch so zu lösen, dass eine universell einsetzbare Strategie entwickelt werden kann, bei der sowohl die Zahl als auch die Art und die gegenseitige Ausrichtung der spezifischen Bindungsmoleküle flexibel ist. Die bisher verwendeten Ansätze zur Herstellung geeigneter Trägerstrukturen beruhen entweder auf der Bildung von Disulfidbrücken³, oder auf dem Einsatz von dendrimerähnlichen Strukturen auf der Basis von Lysin⁴ und stellen daher schon vom Ansatz her eine Einschränkung der Möglichkeiten dar.
³
(a) Nomizu M., Utani A., Shiraishi N., Yamada Y., Roller P.; "Synthesis and conformation of the trimeric coiled-coil segment of laminin"; Int J Pept Protein Res 40(1); 1992; 72-79

⁴
(a) Carrithers M. D., Lerner M. R.; "Synthesis and characterization of bivalent peptide ligands targeted to G-protein-coupled receptors"; Chem Biol 3(7); 1996; 537-542
(b) Wada T., Okada K., Nakamori M., Mochizuki E., Inaki Y.; "Synthesis and properties of oligolysine and oligoglutamic acid derivatives containing nucleosides"; Nucleic Acids Symp Ser 29; 1993; 79-80
(c) Henkel W., Vogl T., Echner H., Voelter W., Urbanke C., Schleuder D., Rauterberg, J.; "Synthesis and folding of native collagen III model peptides"; Biochemistry 38(41); 1999; 13610-22

Der vorliegenden Erfindung liegt die Aufgabe zugrunde, Bindungsmoleküle mit hoher physiologischer Wirksamkeit bereitzustellen, die nicht die aufgeführten Nachteile von Bindungsmolekülen nach dem Stand der Technik aufweisen.

Überraschenderweise wird das der Erfindung zugrunde liegende Problem gelöst durch ein Bindungsmolekül, welches die Benzolthriamid - Struktur aufweist : 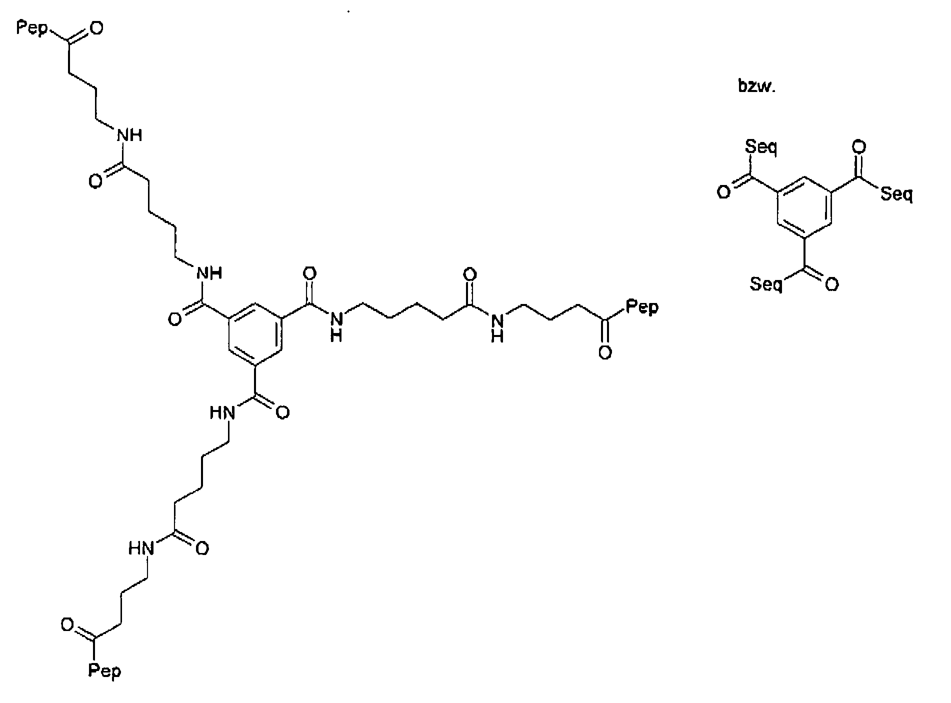 wobei Pep für die Seitenkettenuntereinheiten steht. Die Moleküle umfassen dabei erfindungsgemäß Seitenkettenuntereinheiten, die gleiche Aminosäuresequenz oder unterschiedliche Sequenzen aufweisen.

Die Polypeptidketten der Seitenkettenuntereinheiten bestehen vorzugsweise aus 5 bis 60, vorzugsweise 10 bis 50, insbesondere 12 bis 40 Aminosäuren. Das Molekulargewicht einer solchen Polypeptidketten liegt vorzugsweise nicht über 10 kDa, insbesondere unterhalb 8 kDa oder unterhalb 5 kDa.

In einer bevorzugten Ausführungsform sind die Seitenkettenuntereinheiten mit der Trägerstruktur über Abstandhaltermoleküluntereinheiten (Spacer)/ kovalent verbunden.

Vorzugsweise sind die freien Enden der Seitenkettenuntereinheiten kovalent mit geeigneten Schutzgruppen verbunden, um den Abbau von Exopeptidasen zu verhindern. Die freien Enden können je nach Art der Verknüpfung der Seitenkettenuntereinheiten mit der Trägerstruktur der N - oder C - Terminus sein. Bevorzugte Schutzgruppen sind D-Aminosäuren, vorzugsweise als Di- oder Tripeptid. Weiterhin sind Schutzgruppen verwendbar, die auch im Rahmen der Festphasensynthese üblicherweise verwendet werden, sowie mono- und polymere Kohlehydrate.

Um den Schnitt der Seitenkettenpeptide durch Endopeptidasen zu verhindern, können außerdem die internen Aminosäuren kovalent mit Schutzgruppen verbunden werden. Bevorzugte Schutzgruppen sind Kohlenhydratreste, bevorzugt ausgewählt aus Glucose, Mannose, N-Acetylglucosamin oder N-Acetylgalactosamin, oder Siylinsäure.

Die nicht natürlichen Aminosäuren, die Bestandteil des Bindungsmoleküls der Erfindung sein können, sind in bevorzugten Ausführungsformen solche der Formel NH₂(CH₂)ₙCOOH mit n = 2 bis 8, insbesondere NH₂(CH₂)₂COOH, NH₂(CH₂)₃COOH oder NH₂(CH₂)₄COOH.

Die vorliegende Erfindung macht es möglich, voll synthetische Mimetika physiologische Bindungsmoleküle zu entwickeln und herzustellen, die die geschilderten Nachteile des Standes der Technik überwinden. Anhand der vorliegenden Erfindung werden Trägerstrukturen konzipiert, auf deren Gerüst spezifische Bindungsmoleküle so orientiert werden, dass die Bindungseigenschaften eines biologisch aktiven Bindungsmoleküls nachgebildet werden. Ein großer Vorteil der Erfindung ist die einfache synthetische Herstellung der erfindungsgemäßen Bindungsmoleküle. Die organisch-chemische Synthese ist preiswerter durchführbar und besser kontrollierbar als die rekombinante Herstellung eines analogen Proteinmoleküls. Darüber hinaus können grundsätzlich auch nicht natürliche Aminosäuren, z.B. D-Aminosäuren, oder andere geeignete monomere Bausteine verwendet werden, von denen eine bessere Verträglichkeit, Lebensdauer des Bindungsmoleküls oder Immuntoleranz erwartet werden kann.

Die hier beschriebene Erfindung überwindet die Nachteile des Standes der Technik unter anderem in folgenden Punkten:
a) Sie beschreibt eine einfach darstellbare zyklische Trägerstuktur, die im allgemeinen leicht herstellbar und verfügbar ist, und die für die Positionierung spezifischer Bindungsmoleküle eingesetzt werden kann.
b) Die zyklische Struktur der Trägerstruktur erlaubt über die Ringgröße und die Abstände zwischen den Stellen, an denen die Seitenkettenuntereinheiten gebunden sind, sowie gegebenenfalls durch den Einbau geeigneter Abstandshaltermoleküle die Positionierung spezifischer Bindungsmoleküle in praktisch jeder gewünschten räumlichen Orientierung. Die erfindungsgemäßen Bindungsmoleküle unterscheiden sich damit deutlich von solchen nach dem Stand der Technik, bei denen lediglich eine zufällige Vernetzung von Peptiden mit synthetischen Molekülen vorgenommen wurde, und bei denen eine definierte räumliche Ausrichtung der Molekülbestandteile nicht möglich ist.
c) Durch Wahl geeigneter monomerer Bausteine kann die zyklische Trägerstruktur in definierter Sequenz an Festphase synthetisiert und die spezifischen Bindungsmoleküle als Seitenkettenuntereinheiten montiert bzw. synthetisiert werden. Im Falle von Aminosäuren als monomeren Bausteine ist so auch die komplette Festphasensynthese von parallel oder antiparallel orientierten Peptiden an der Trägerstruktur möglich.
d) Die Trägerstruktur ist flexibel im Hinblick auf Art und Länge der zu montierenden spezifischen Bindungsmoleküle als Seitenkettenuntereinheiten.
e) Erfindungsgemäß können parallel und antiparallel orientierte Aminosäuresequenzen in einem Synthesegang an Festphase an die Trägerstruktur gekoppelt und gegebenenfalls gemeinsam mit ihr in einem Arbeitsgang synthetisiert werden. Letzteres ist für Ausbeute und Reinheit des Produktes sowie den Preis der Herstellung von Vorteil. Unter paralleler Ausrichtung wird hier verstanden, dass die Seitenkettenuntereinheiten alle gleichsinnig mit dem N- oder C- terminalen Ende an die Trägerstruktur gebunden sind. Unter antiparalleler Ausrichtung wird hier verstanden, dass mindestens eine Seitenkettenuntereinheit N-terminal und mindestens eine C-terminal mit der Trägerstruktur verbunden ist.

Durch die Verwendung von nicht-natürlichen Aminosäuren und D-Aminosäuren kann die Flexibilität bei der geplanten Positionierung spezifischer Bindungsmoleküle erhöht werden. Die Verwendung von nicht natürlichen und D-Aminosäuren bzw. von Lactonen und Lactamen erhöht die Stabilität gegen proteolytischen Abbau. Durch die Variabilität der Trägerstrukturen lassen sich zusätzliche Funktionalisierungen, die die Stabilität bzw. Halbwertszeit erhöhen, einführen. Hierunter fallen z.B. Glycosylierungen, Acetylierungen oder andere chemische Derivatisierungen, wie sie dem Fachmann aus der Schutzgruppenchemie bekannt sind.

Handelt es sich bei den auf der erfindungsgemäßen Trägerstruktur zu positionierenden spezifischen Bindungsmolekülen um Peptide, so sind diese an ein und demselben Trägergerüst parallel und antiparallel orientierbar und synthetisierbar.

Eine bevorzugte Ausführungsform für die erfindungsgemäßen Trägerstrukturen sind zyklische Peptide. Für zyklische Peptide sind geeignete Schutzgruppenstrategien nach dem Stand der Technik verfügbar. Zyklische Peptide werden in vivo langsamer abgebaut, da diese nur von Endopeptidasen gespalten werden können. Nach dem erfindungsgemäßen Verfahren sind sie darüber hinaus durch Einbau nicht natürlicher Aminosäuren, z.B. von D-Aminosäuren, proteolyseresistenter darstellbar.

### Interleukin-2-Rezeptor spezifische Bindungsmoleküle

In einer Ausführungsform der Erfindung werden synthetische Bindungsmoleküle bereitgestellt, die in Analogie zum Interleukin-2 (IL2) die Fähigkeit aufweisen, mit hoher Affinität an IL2 spezifische Rezeptoren zu binden. IL2 ist ein in der Tumortherapie eingesetztes Zytokin. Es handelt sich um ein Protein bestehend aus 133 Aminosäuren mit einem Molekulargewicht von 15.4 kDa. IL2 bindet an spezifische Rezeptoren (IL2R), wodurch die IL2-spezifischen intrazellulären Signale ausgelöst werden. Der hochaffine Rezeptor ist ein Rezeptorkomplex bestehend aus den Untereinheiten a, b und g (jeweils auch bezeichnet als p55, p75 und p64). Die Stimulation des aus p75 und p64 bestehenden Rezeptorkomplexes reicht aus, um die volle IL2R Aktivität zu induzieren.

IL2 hat stimulierende Wirkungen auf das Wachstum von T- und B-Lymphozyten, aktiviert cytotoxische und cytolytische NK- Zellen und hat dadurch zentrale Bedeutung bei der Regulation der Immunantwort. Damit spielt IL2 eine fundamentale Rolle bei der Immunantwort gegen Tumore sowie bei entzündlichen Reaktionen. Einer der Mechanismen, der bei der Tumorabwehr durch IL2 Bedeutung hat, scheint die Induktion von LAKs ("Lymphokine Activated Killer Cells") zu sein. Diese Zellen sind imstande, Tumorzellen abzutöten.

In den vergangenen Jahren wurden vielfältige Anläufe bei der Tumortherapie mit IL2 unternommen. Inzwischen befindet sich ein rekombinant hergestelltes Produkt auf dem Markt (Aldesleukin von Chiron) zur Therapie von metastasierendem Nierenzellcarcinom und metastatischem Myelom. Die rekombinante Herstellung hat hohe Entwicklungs-, Zulassungs- und Produktionskosten und dementsprechend hohe Marktpreise zur Folge. Darüber hinaus sind rekombinante Herstellungsprozesse in hohem Masse störanfällig. Nur ca. 30% der behandelten Patienten zeigen eine Reaktion auf die IL2 - Therapie. Die breite klinische Anwendung von IL2 wird durch die extrem kurze Halbwertszeit im menschlichen Organismus (weniger als 10 Minuten) und durch eine breite Toxizität verhindert, wobei die Mortalitätsrate in einer Studie bei 4% lag.

Neben Übelkeit, Erbrechen und Durchfall treten vor allem Organdisfunktionen und neuropsychiatrische Effekte bei der Therapie mit IL2 auf. Die Toxizität ist dermaßen hoch, dass nur 10% der im Tierversuch als optimal ermittelten Dosis beim Menschen zum Einsatz kommt. Damit kann das therapeutische Potential dieses Zytokins nicht voll ausgeschöpft werden.

Mitunter reicht die Verabreichung einer kurzen IL2-Domäne, die mit dem Rezeptor interagiert, bereits aus, um zumindest einige der zellulären Effekte (z. B. immunstimulatorische Wirkung) zu erzielen. In einer Studie konnte gezeigt werden, dass ein IL2-Peptid der Aminosäuresequenz 1-30 IL2-ähnliche Wirkungen hat, allerdings nur in wesentlich höheren Konzentrationen⁵. Dies dürfte darauf zurückzuführen sein, dass dieses Peptid nur an die β-Kette des Rezeptorkomplexes bindet und damit nur den mittelaffinen Rezeptorkomplex aktiviert. Ähnliches gilt für andere beschriebene Sequenzen aus einem anderen IL2- Bereich⁶. Alle diese Peptide wurden jedoch ohne die gezielte Montage auf einer Trägerstruktur synthetisiert, die die vorliegende Erfindung charakterisiert. Sie können daher nicht zur Rezeptordimerisierung beitragen und wirken ausschließlich über mittel- bis niedrig affine Rezeptoruntereinheiten. Ihre Wirkung ist daher schwach. Diese Peptide sind außerdem nicht gegen proteolytischen Abbau durch Einbau von Schutzgruppen geschützt worden. Sie unterliegen damit in vivo einem beschleunigten Abbau.
⁵ Eckenberg R, Xu D, Moreau J, Bossus M, Mazie J, Tartar A, Liu X, Alzari P, Bertoglio J, Theze J (1997) Analysis of human IL-2/IL-" Receptor beta chain interactions: Monoclonal antibody H2-8 and new IL-2 Mutants define the critical role of alpha Helix-A of IL-2. Cytokine 9:488-498
⁶ Eckenberg R, Rose T, Moreau J-L, Weil R, Gesbert F, Dubois S, Tello D, Bossus M, Gras H, Tartar A, Bertoglio J, Chouaib S, Goldberg M, Jacques Y, Alzari PM, Thèze J (2000) The first α-Helix of Interleukin(IL)-2 Folds as a Homotetramer, Acts as an Agonist of the IL-2 Receptor β Chain, and Induces Lymphokine -activated Killer Cells. J. Exp. Med. 3:529-39
Berndt W, Chang D, Smith K, Ciardelli T (1994) Mutagenic analysis of a receptor contact site on interleukin 2: Preparation of an Interleukin-2 analog with increased potency. Biochemistry 33:6571-6577

Die vorliegende Erfindung erlaubt die Bereitstellung von synthetischen IL2R-spezifischen Bindungsmolekülen mit hoher Wirksamkeit, die die Nachteile bei der Herstellung und Verwendung von IL-2 oder Peptidfragmenten von IL-2 vermeidet.

Dabei weisen die Seitenkettenuntereinheiten vorzugsweise mindestens eine der Sequenzen
• APTSSSTKKT₁ QLQLEHX¹X²X³X⁴ X⁵QMILNGINN oder
• TIVX⁶FLNRWIT FX⁷QSX⁸ISTLT₁ auf,
wobei
X¹ ausgewählt ist aus I oder L,
X² ausgewählt ist aus I oder L,
X³ ausgewählt ist aus V, L oder M,
X⁴ ausgewählt ist aus E, D oder K,
X⁵ ausgewählt ist aus L oder F,
X⁶ ausgewählt ist aus E oder D,
X⁷ ausgewählt ist aus A, G oder C,
X⁸ ausgewählt ist aus A oder I; und
an der Position T₁ zum Schutz vor proteolytischem Abbau eine Schutzgruppe nach dem Stand der Technik, bevorzugt ein Kohlenhydratrest, bevorzugt ausgewählt aus Glucose, Mannose, N-Acetylglucosamin oder N-Acetylgalactosamin auf geeignete Weise kovalent an Threonin gebunden sein kann. Erfindungsgemäß verwendbar sind auch Bindungsmoleküle, bei denen die Seitenkettenuntereinheiten zu den angegebenen Sequenzen zu mindestens 80%, vorzugsweise 90 oder 95% Sequenzhomologie aufweisen. Die Sequenzabweichungen sind dabei insbesondere konservative Aminosäurenaustausche.

Bevorzugte Bindungsmoleküle sind solche mit dem Aufbau:
• APTSSSTKKT₁ QLQLEHX¹X²X³X⁴ X⁵QMILNGINN *-Trägerstruktur-*TIVX⁶FLNRWIT FX⁷QSX⁸ISTLT₁;
• TIVX⁶FLNRWIT FX⁷QSX⁸ISTLT, *- Trägerstruktur-* APTSSSTKKT₁ QLQLEHX¹X²X³X⁴ X⁵QMILNGINN;
• APTSSSTKKT₁ QLQLEHX¹X²X³X⁴ X⁵QMILNGINN *-Trägerstruktur-*APTSSSTKKT₁ QLQLEHX⁰¹X⁰²X⁰³X⁰⁴ X⁰⁵QMILNGINN oder
• TIVX⁶FLNRWIT FX⁷QSX⁸ISTLT₁ *-Trägerstruktur-* TIVX⁰⁶FLNRWIT FX⁰⁷QSX⁰⁸ISTLT₁
wobei die Aminosäuren X⁰¹ ,X⁰² ,X⁰³, X⁰⁴ und X⁰⁵ ausgewählt werden wie für X¹, X² ,X³ ,X⁴ und X⁵ angegeben. Erfindungsgemäß verwendbar sind auch Bindungsmoleküle, bei denen die Seitenkettenuntereinheiten zu den angegebenen Sequenzen zu mindestens 80%, vorzugsweise 90 oder 95% Sequenzhomologie aufweisen. Die Sequenzabweichungen sind dabei insbesondere konservative Aminosäurenaustausche.

Durch die IL2R-spezifischen Bindungsmoleküle können die wesentlichen Nachteile des Standes der Technik überwunden werden. Die Bindungsmoleküle können einfach und preisgünstig hergestellt werden. Die hohen Herstellungskosten und die physiologische Instabilität der rekombinanten Moleküle treten nicht auf, die bei der Verwendung von Peptiden beobachtete unzureichende Stimulation der Rezeptoren tritt nicht auf, da die erfindungsgemäßen Bindungsmoleküle funktionell kooperativ in einer definierten räumlichen Anordnung an den Rezeptorkomplex binden, wobei die Trägerstruktur keine Affinität zum Rezeptor aufweist. Die erfindungsgemäßen Rezeptormoleküle bieten auch die Möglichkeit, durch gezielte Sequenzoptimierung die Wirkung zu verbessern und Nebenwirkungen zu minimieren.

Die oben aufgeführten synthetischen IL2R-spezifischen Bindungsmoleküle eignen sich zur Behandlung von Erkrankungen des Immunsystems, beispielsweise Entzündungen und arthritischen Prozessen oder von Immundefizienzsyndromen aller Art und Genese; Erkrankungen, die mit einer erhöhten Zellproliferation in Zusammenhang stehen, beispielsweise Krebserkrankungen, beispielsweise Formen von Carzinomen, Sarkomen, Lymphomen und Leukämien; oder infektiösen Prozessen. Es ist bereits bekannt, dass rekombinantes Interleukin 2 in Kombination mit Interleukin 12 positive Effekte bei der Therapie von Tumoren hat. Zusätzlich können auch solche Agenzien als bevorzugte Kombinationsstoffe zum erfindungsgemäßen Arzneimittel kombiniert werden, die Apoptose in Zielzellen, insbesondere in Tumorzellen auslösen. Der erwartete positive Effekt auf den therapeutischen Erfolg kommt hier dadurch zustande, dass lokale Apoptose im Tumor und generelle Immunstimulation durch ein erfindungsgemäßes Arzneimittel eine lokale Steigerung der Effektivität bedingen. Besonders bevorzugt ist es dabei, TRAIL (TNF-Related Apoptosis Inducing Ligand) Rezeptor spezifische Bindungs- und Effektormoleküle einzusetzen.

### TRAIL-Rezeptor spezifische Bindungsmoleküle

TRAIL *(TNF-related apoptosis-inducing ligand*) ist ein zur TNF-Familie gehöriges Protein, das in Tumorzellen selektiv den programmierten Zelltod (*Apoptose*) auslöst. Die hier, bevorzugt auf Basis geeigneter zyklischer Trägerstrukturen synthetisierten, TRAIL-spezifischen Bindungs- und Effektormoleküle binden spezifisch an die von Tumorzellen exprimierten TRAIL-Rezeptoren und lösen Apoptose aus. Proteine, die Apoptose auslösen, sind seit längerem bekannt, wie der TNF (*Tumor-Nekrose-Faktor*) oder Fas und APO1. Das Cytokin TNF wurde ursprünglich als ein Protein charakterisiert, das in Mäusen eine starke tumorizide Wirkung hatte. Allerdings konnte es beim Menschen aufgrund seiner systemischen Toxizität nicht therapeutisch eingesetzt werden. Dasselbe gilt für Fas und APO1. 1999 wurde erstmals TRAIL beschrieben⁷. Dieses Protein besteht aus 281 Aminosäuren, ist ein Typ-II-Membranprotein, das durch Abspaltung in ein lösliches Molekül bestehend aus den Aminosäuren 114-281 überführt wird. Nach spontaner Trimerisierung bindet es an seine spezifischen Rezeptoren, induziert dadurch Trimerisierung der Rezeptoren und löst so das intrazelluläre, Caspase-vermittelte Programm zum Zelltod aus. Für TRAIL sind inzwischen 4 verschiedene Rezeptoren beschrieben: TRAIL-R1 und 2, die beide an der Signalvermittlung beteiligt sind, sowie die beiden Rezeptoren TRAIL R3 und 4, die jedoch keine sog. "death domain" besitzen und somit nach Bindung von TRAIL keine Signale auslösen können. Sowohl TRAIL selbst als auch die erwähnten Rezeptoren werden in den meisten menschlichen Geweben und Organen exprimiert; die selektive tumorizide Wirkung beruht deshalb wahrscheinlich darauf, dass normale Zellen mehr von den "decoy-Rezeptoren" 3 und 4 besitzen, die eine effiziente Signalauslösung durch Hemmung der Trimerisierung der aktiven Rezeptoren verhindern.
⁷ Walczak, H. et al. Nature Medicine 5 (1999) 157-163

In der Aminosäuresequenz des natürlichen oder rekombinant hergestellten TRAIL gibt es verschiedene Domänen, die für die Bindung an den Rezeptor und damit für die biologische Aktivität verantwortlich sind, und zwar sind das die Domänen AA131-163, AA201-205, AA214-220, AA237-240, AA258-283. Diese kurzen, in sterischer Nachbarschaft positionierten Domänen sind kooperativ für die Interaktion mit dem TRAIL Rezeptor verantwortlich.

Die vorliegende Erfindung ermöglicht die Bereitstellung von Bindungsmolekülen, die TRAIL-Rezeptoren spezifisch mit hoher Affinität binden. Um die biologische Wirkung zu erzielen, werden in erfindungsgemäßen Bindungsmolekülen die den oben erwähnten Abschnitten analoge Domänen in geeigneter kooperativer Anordnung an zyklische Trägerstrukturen gekoppelt.

Die erfindungsgemäßen TRAILR-Bindungsmoleküle liegen in bevorzugten Ausführungsformen in trimerisierter Form vor. Durch die kovalente Bindung an eine zyklische Moleküluntereinheit besitzen sie eine hohe biologische Halbwertszeit und durch Trimerisierung die biologisch höchst-mögliche Wirksamkeit mit gleichzeitig minimalen Nebenwirkungen. Die Peptide stellen die minimale Struktur von TRAIL dar, die für seine biologische Wirksamkeit erforderlich ist. Dadurch wird höchste Spezifität mit geringsten Nebenwirkungen erreicht. Die Trimere können in bevorzugten Ausführungsformen entweder homotrivalent über eine zyklische Trägerstruktur oder aber über einen Benzoltriamidlinker dargestellt werden.

Vorteilhafterweise können die TRAIL-Peptide in einer Saccharid-modifizierten Form hergestellt, so dass sie eine hohe biologische Halbwertszeit aufweisen. Dadurch reduzieren sich die Applikationshäufigkeit und -menge damit einhergehend die Belastung für den Patienten. Durch gezielten Aminosäure-Austausch können die Peptide zum einen in eine biologisch noch wirksamere Form oder eine Form mit erhöhter biologischer Halbwertszeit überführt werden, zum anderen in eine antagonistische - also hemmende - Form.

In einer Ausführungsform weist mindestens eine Seitenkettenuntereinheit des TRAILR-spezifischen Bindungsmoleküls folgende Sequenz auf:
SKNEKALGRKINSWESSRSGHSFLS

In besonderen Ausführungsformen weisen die Bindungsmoleküle mit Affinität für TRAIL-Rezeptoren mindestens eine Seitenkettenuntereinheit der Sequenz
SKNEKALGRKIX₁X₂X₃X₄SSRX₅GHSFLS, mit
X₁= N oder L
X₂= S oder Q oder L oder E
X₃= W oder L oder R oder A oder Y
X₄= E oder N oder A oder D oder H
X₅= S oder A auf.

Gegenstand der Erfindung sind auch Bindungsmoleküle, bei denen die Seitenkettenuntereinheiten zu den angegebenen Sequenzen zu mindestens 80%, vorzugsweise 90 oder 95% Sequenzhomologie aufweisen. Die Sequenzabweichungen sind dabei insbesondere konservative Aminosäurenaustausche.

Besondere Ausführungsformen sind solche der Strukturformeln

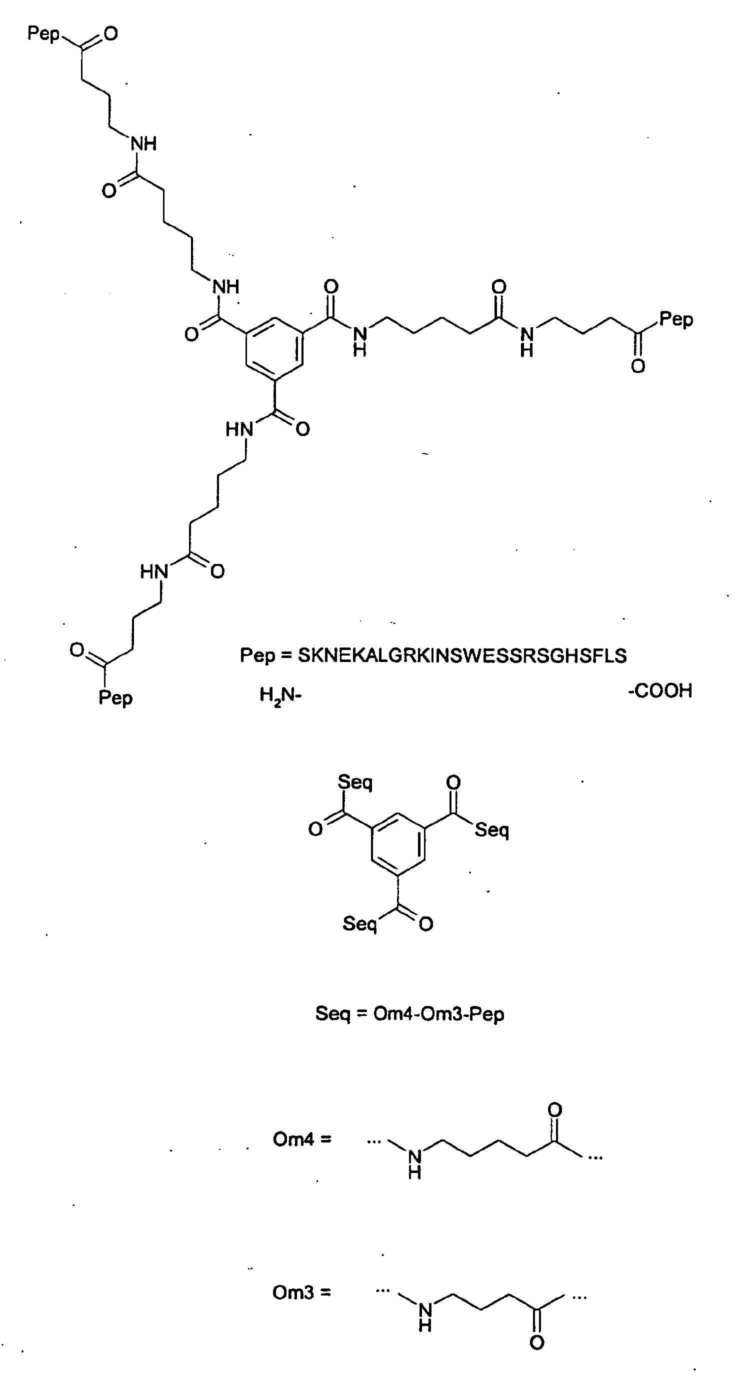

Erfindungsgemäß verwendbar sind auch Bindungsmoleküle, bei denen die Seitenkettenuntereinheiten zu den angegebenen Sequenzen zu mindestens 80%, vorzugsweise 90 oder 95% Sequenzhomologie aufweisen. Die Sequenzabweichungen sind dabei insbesondere konservative Aminosäurenaustausche.

### Antikörper und ihre Antiidiotype

Eine weitere wichtige, biologisch aktive Substanzklasse, die für die hier vorliegende Erfindung relevant ist, sind Antikörper. Antikörper sind körpereigene Stoffe, die eine spezifische Bindung an in der Regel körperfremde Stoffe, die als Antigene bezeichnet werden, aufweisen. Antikörper spielen eine wichtige Rolle bei der Abwehr von Pathogenen und beim Schutz des Organismus vor Infektionen. In vielen therapeutischen und diagnostischen Anwendungen werden Antikörper eingesetzt oder entwickelt, die eine für die geplante Indikation relevante Bindungsfähigkeit haben. So werden Antikörper eingesetzt, um Zytostatika oder andere tumorschädigende Agentien spezifisch in Tumoren anzureichern oder sie werden benutzt, um bestimmte Körperregionen oder Erkrankungsbereiche in bildgebenden Verfahren darzustellen, indem sie mit Kontrastmittel beladen werden. Antikörper können als natürliche Antikörper aus dem menschlichen Körper isoliert werden. Sogenannte monoklonale Antikörper sind im Regelfalle Antikörper, die durch Immunisierung von Labornagern in diesen erzeugt wurden und dann durch Fusion antikörperproduzierender Zellen mit Myelomzellen immortalisiert und durch Vereinzelung monoklonalisiert werden. Darüber hinaus können Antikörper auch durch rekombinante Techniken monoklonalisiert werden, indem die sie codierenden genetischen Informationen in geeignete, oftmals bakterielle oder auf Hefe basierende Expressionssysteme transferiert werden. Rekombinant hergestellte Antikörper können aus allen Spezies gewonnen werden, deren Immunglobulin-Gen- Repertoire ausreichend sequenziert und bekannt ist. Im folgenden soll daher gemäß obiger Beschreibung von natürlich vorkommenden, monoklonalen oder rekombinant hergestellten Antikörpern die Rede sein.

Antikörper sind relativ große Moleküle, deren spezifischer Bindungsbereich im Regelfalle 6 hypervariable Bereiche (bezogen auf die Aminosäuresequenz) enthält. Über diese sogenannten CDR ("Complementarity Determining Region") Bereiche werden die Bindungseigenschaften eines Antikörpers definiert. In vielen Fällen sind jedoch nicht alle 6 CDR-Bereiche kooperativ an der Bindung beteiligt, sondern sind einzelne oder mehrere Bereiche nicht in kritischer Weise für die spezifische Bindung erforderlich. Sowohl monoklonale Antikörper als auch rekombinant hergestellte Antikörper werden in vielfältigen Indikationen therapeutisch oder diagnostisch eingesetzt. Bei der therapeutischen Anwendung von Antikörpern ergeben sich jedoch eine Reihe von Nachteilen. Zum einen ist die Herstellung von Antikörpern auf beiden Wegen (monoklonal und rekombinant) aufwändig und sehr teuer. Zum anderen sind Antikörper selbst natürliche Moleküle, auf die das Immunsystem des Patienten reagiert. Die Therapie kann durch Produktion patienteneigener Antikörper (gegen den therapeutischen Antikörper) oder durch anaphylaktische Reaktionen wesentlich erschwert werden. Um diese Probleme zu umgehen, müssen die Herstellungsverfahren für die therapeutischen Antikörper verändert werden und z.B. monoklonale Antikörper der Maus in einem molekularbiologischen, zeit- und kostenaufwendigen Prozess in die rekombinante Herstellung überführt und gleichzeitig auf diesem Wege humanisiert, d.h. durch Einbau menschlicher Sequenzen für therapeutischen Einsatz optimiert werden.

Nach dem Stand der Technik ist es bereits jetzt möglich, anti-idiotypische Substanzen aus Bibliotheken, z.B. rekombinanten Peptidbibliotheken, zu isolieren. In der Regel binden die so isolierten Peptide jedoch nur mit mäßiger Affinität an den Antikörper und sind nicht oder nur eingeschränkt in der Lage, die pathologische Antikörperbindung kompetitiv zu unterbinden.

Die vorliegende Erfindung löst die aufgeführten Probleme durch Bereitstellung von synthetischen Bindungsmolekülen, bei denen die Bindungseigenschaften eines therapeutisch relevanten Antikörpers nachgebildet werden. Mit der erfindungsgemäßen Trägerstruktur ist es möglich, die eben geschilderten Nachteile des Standes der Technik zu überwinden und vollsynthetische Mimetika der betreffenden Antikörper zu entwickeln und zu konstruieren. Die organisch-chemische Synthese ist preiswerter durchführbar und besser kontrollierbar als die rekombinante Herstellung eines analogen Moleküls. Darüber hinaus können grundsätzlich auch nicht natürliche Aminosäuren oder andere geeignete monomere Bausteine verwendet werden, von denen eine bessere Verträglichkeit, Lebensdauer oder Immuntoleranz erwartet werden kann.

Antikörper können jedoch nicht nur therapeutisch von Bedeutung sein, sondern auch pathogenetische Reagentien darstellen. So sind Antikörper pathologisch, wenn sie sich gegen körpereigene Gewebe richten und damit eine sogenannte Autoimmunerkrankung auslösen oder zumindest mit ihr vergesellschaftet sind. Eine weitere Variante der pathologischen Rolle von Antikörpern oder anderen Substanzen der Immunglobulinsuperfamilie ist bei der Exposition von allergenen Substanzen bei Patienten gegeben, die gegen die betreffende Substanz allergisch sind. Auch diese Erkrankungen sind Fehlreaktionen des immunologischen Apparates und ihre Auslösung wird durch Bindung des betreffenden Allergens ausgelöst. In allen diesen Fällen wäre es wünschenswert, die pathogenetisch relevanten Antikörper dadurch zu neutralisieren, dass sie mit sogenannten anti-idiotypischen Substanzen reagieren. Unter Anti-idiotypisch ist dabei zu verstehen, dass die Substanz wie das eigentliche Antigen spezifisch an die Bindungsgrube des Antikörpers bindet. Dadurch wird die pathologische Bindung an körpereigene Antigene verhindert.

Gegenstand der Erfindung ist auch das Verfahren zur Herstellung eines Bindungsmoleküls, das als Festphasensyntheseverfahren durchgeführt wird, bei der das Peptid der Seitenkettenuntereinheit an der Festphase gebunden sukzessive mit den entsprechenden Aminosäuren verlängert wird, wobei sich gegebenenfalls im letzten Schritt eine Ankopplung an die Trägerstruktur anschließt.

Gegenstand der Erfindung sind auch Arzneimittel und Diagnostikmittel, die die erfindungsgemäßen Bindungsmoleküle oder Peptide enthalten.

Die Verwendung von erfindungsgemäßen Bindungsmolekülen erfolgt vorzugsweise zur Herstellung eines Arzneimittels oder Diagnostikmittels zur Behandlung oder zum Nachweis von Erkrankungen des Immunsystems, insbesondere Entzündungen, arthritische Prozesse, Immundefizienzsyndrome, Autoimmunsyndrome und Immunschwächesyndrome, Fertilitätsstörungen, zur Kontrazeption oder antiviralen Prophylaxe, Erkrankungen, die mit einer erhöhten Zellproliferation in Zusammenhang stehen, insbesondere Tumorerkrankungen, Krebserkrankungen, Carcinomen, Sarkomen, Lymphomen und Leukämien; und/oder infektiösen Prozessen beim Menschen.

Das erfindungsgemäße Arzneimittel wird insbesondere in Form einer Mikroverkapselung, liposomalen Zubereitung oder Depotpräparation, enthaltend geeignete Zusatz-, Träger-, Hilfsstoffe und/oder mindestens einen weiteren Wirkstoff, bereitgestellt. Das erfindungsgemäße Arzneimittel liegt gegebenenfalls in Kombination mit einem üblichen geeigneten pharmazeutischen Träger vor. Zu geeigneten Trägern zählen beispielsweise gepufferte Kochsalzlösungen, Wasser, Emulsionen, beispielsweise Öl/Wasser-Emulsionen, Netzmittel, sterile Lösungen etc. Das erfindungsgemäße Arzneimittel kann in Form einer Injektionslösung, Tablette, Salbe, Suspension, Emulsion, eines Zäpfchens, eines Aerosols etc. vorliegen. Es kann auch in Form von Depots (Mikrokapseln, Zinksalze, Liposomen etc.) verabreicht werden. Aufgrund der geringen Größe der Peptide können sie mikroenkapsuliert werden, wodurch sich Depotformen mit - abhängig von der jeweiligen Porengröße der Kapseln - unterschiedlich langer Wirkdauer ergeben. Die Depotform kann lokal appliziert werden, so daß an der gewünschten Stelle (z. B. Krebsherd) die höchste Konzentration über einen langen Zeitraum gewährleistet ist. Die Art der Verabreichung des Arzneimittels hängt unter anderem davon ab, in welcher Form der Wirkstoff vorliegt, sie kann oral oder parenteral erfolgen. Die einzelnen Verfahren sind dem Fachmann bekannt. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, der Art der Verabreichung etc.

In einer bevorzugten Ausführungsform erfolgt der Einsatz eines erfindungsgemäßen Bindungsmoleküls, insbesondere IL2-spezifisch, indem dieses zur Präparation humaner LAK (Lymphokine Activated Killer Cells) *ex vivo* eingesetzt wird. Bei einer solchen *ex vivo* Therapie werden Zellen des Patienten entnommen, in der Kulturschale mit dem Wirkstoff behandelt und aktiviert und anschließend dem Patienten wieder reinfundiert.

In besonderen Ausführungsformen der Erfindung wird als weiterer Wirkstoff Interleukin 12, ein Interleukin 12 Rezeptor spezifisches Bindungs- und Effektormolekül oder rekombinantes IL12 verwendet.

In einer weiteren Ausführungsform wird als weiterer Wirkstoff TRAIL (TNF-related apoptosis inducing ligand), ein TRAIL-Rezeptor spezifisches Bindungs- und Effektormolekül oder rekombinant hergestelltes TRAIL verwendet.

In einer besonderen Ausführungsform wird dem erfindungsgemäßen Arzneimittel als weiterer Wirkstoff mindestens ein Virostatikum zugesetzt, insbesondere ein Virostatikum, das imstande ist, den Eintritt von Viruspartikeln, insbesondere HIV-Partikeln, in Zielzellen, insbesondere T-Lymphozyten, zu verlangsamen oder zu blockieren.

### Beispiel 1:

### Multimerisierungsprinzip von Peptiden auf der Basis von Cyclopeptiden

### A: antiparallele Dimere:

Ein cyclisches Peptid wird generiert und als Ausgangspunkt für die Festphasensynthese eingesetzt. Dabei wird zunächst der erste Peptidstrang an der Festphase synthetisiert, dann das Cyclopeptid angekoppelt und anschließend der zweite Peptidstrang entweder an diesem beladenen Harz Schritt für Schritt synthetisiert oder das vorher gereinigte Peptid B angekoppelt

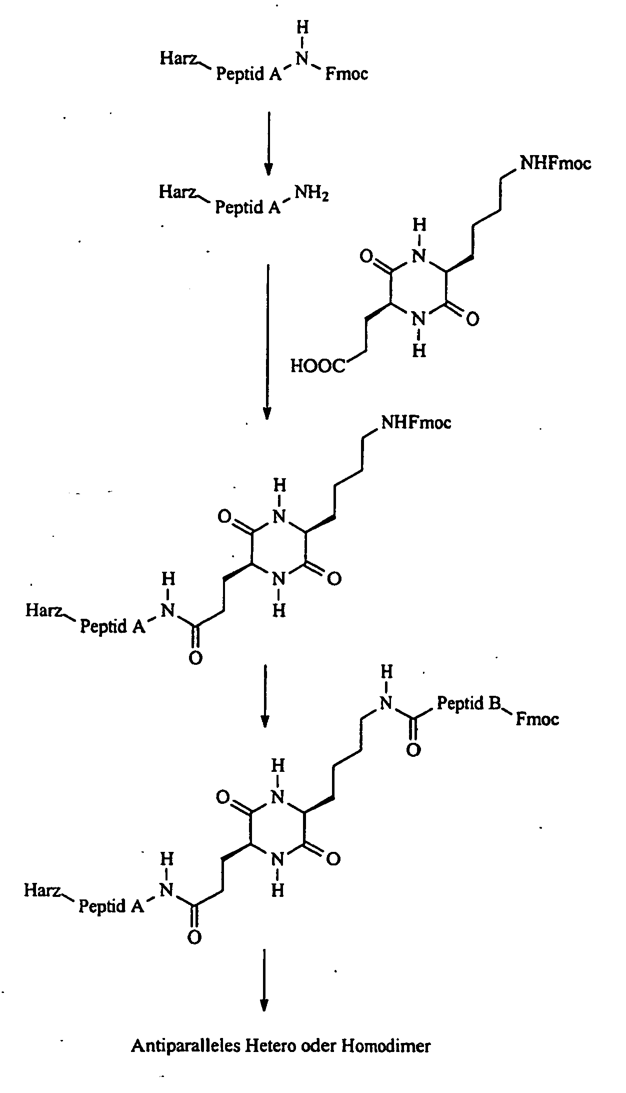

### Kopplung von Lysin-Cyclopeptiden und Asparaginsäure in Lösung

Zu einer auf 0°C gekühlten Lösung von 1mmol des Asparaginsäurederivates in 10ml Methylenchlorid gibt man unter Argonatmosphäre 0.2ml NMM sowie 1mmol CDMT und rührt diese Mischung für 2 Stunden bei 0°C. Anschließend gibt man weitere 0.2ml NMM sowie 1 Moläquivalent des Cyclopeptides in 10ml absolutem DMF hinzu. Die Mischung wird 2h bei 0°C und über Nacht bei RT gerührt, mit 50ml Eiswasser versetzt und zweimal mit je 100ml Ethylacetat extrahiert. Die vereinigten organischen Phasen werden mit gesättigter Natriumhydrogencarbonatlösung und mit 5%iger Natriumhydrogensulfatlösung ausgeschüttelt. Nach Trocknen über Natriumsulfat wird die verbleibende Lösung am Rotavapor eingeengt und der Rückstand in Methylenchlorid aufgeschlämmt, wobei das Produkt als weißer Feststoff anfällt.

**Ansatzgröße:**

| | | |
|---|---|---|
| Boc-L-Asp(OBz) | 162mg | 0.5mmol |
| cyclo-L-Ala-L-Lys | 100mg | 0.5mmol |
| CDMT | 89mg | 0.5mmol |
| NMM | 0.2ml | |
| Methylenchlorid | 5ml | |
| DMF | 5ml | |

**Ausbeute :**

| | |
|---|---|
| Theoretisch | 0.25g 100% |
| Real | 0.18g 72% |

### B: parallele Dimere

Ein Cyclohexapeptid des Sequenz D-K-D-K-D-K wird gemäß des unten stehenden Schemas generiert. Durch geeignete Wahl der Schutzgruppen kann das Cyclopeptid über die Seitenkette einer der Asparaginsäuren an einen polymeren Träger evtl. über Abstandhaltermoleküle gebunden werden kann und drei Peptidstränge können dann parallel am Harz synthetisiert werden, oder die gereinigten Peptide können an Festphase angekoppelt werden.

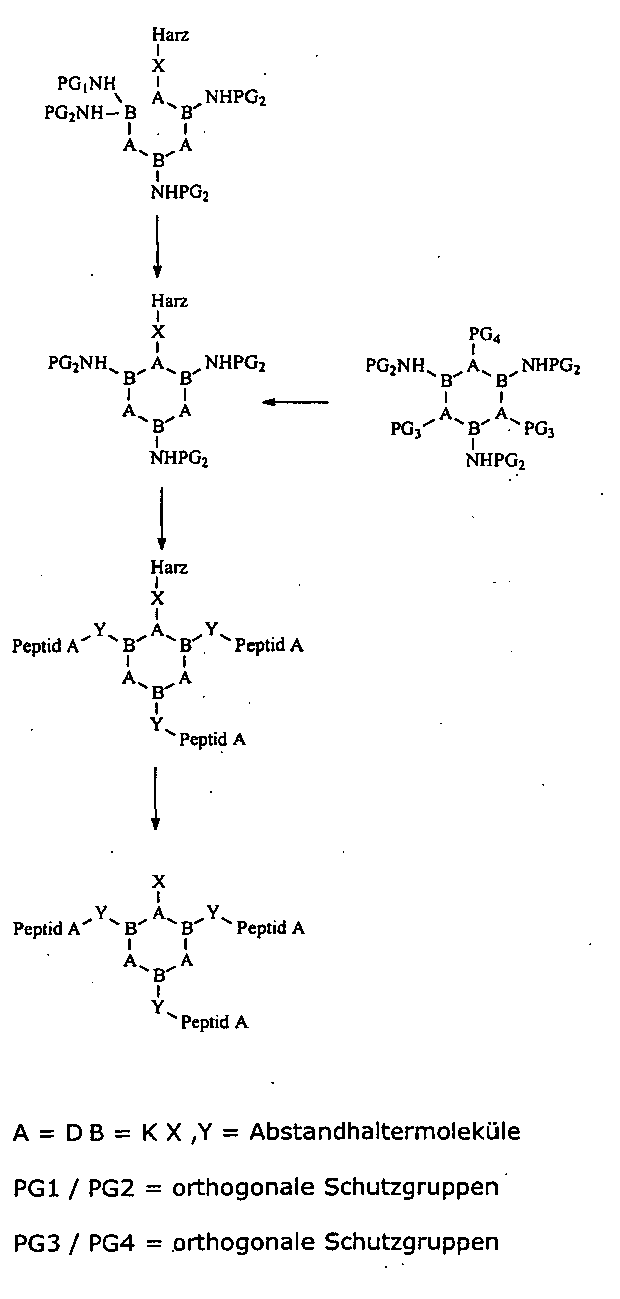

### Beispiel 2

### Ausführungsbeispiele für Peptid A gemäß Beispiel 1B in der Form von IL2R spezifischen Bindungsmolekülen:

Die Peptide werden wie folgt synthetisiert:
Synthese: Mittels Festphasensynthese (100mg Wang-Harz (0.4mmol/g) je Ansatz) werden auf einem Syntheseroboter (Sophas-3, Zinsser Analytik) Peptide variabler Sequenzen automatisiert hergestellt. Als Monomerbausteine dienen Fmoc-derivatisierte Aminosäuren (c=0.5 mol/l), mit geeigneten weiteren Schutzgruppen nach dem Stand der Technik. Die Aminosäurebausteine, sowie Kopplungsreagenzien (HOBt c=0,766 g/ml, PyBOP c=0,2602 g/ml) lagen in DMF gelöst vor. Die einzelnen Kopplungsschritte wurden für je 2x 45 min durchgeführt und das Harz wurde zwischen den Schritten mit DMF gewaschen. Die Fmoc-Abspaltung erfolgte durch 20 min Behandlung mit 50%(v/v) Piperidin in DMF. Die anschließende Abspaltung vom Harz erfolgte in Gegenwart eines Gemisches aus 95% Trifluoressigsäure, 2,5% Wasser, 2,5% Diisopropylsilan für 20 min. Die abgespaltenen Peptide wurden durch Versetzen mit tert-Butyl-Methyl-Ether ausgefällt, abzentrifugiert und erneut mit tert-Butyl-Methyl-Ether versetzt und abzentrifugiert. Anschließend wurden die Peptide lyophylisiert und mittels Reversed-Phase HPLC aufgereinigt und per LC/MS (Thermoquest LCQDuo) charakterisiert. Die gereinigten Peptide wurden in Zellkultur auf deren Zytotoxizitäts-steigernde Wirkung hin überprüft.

Es wurden die Peptide mit den Sequenzen

| | |
|---|---|
| Sequenz 1: | APTSSSTKKT QLQLEHLLLK LQMILNGINN |
| Sequenz 2 | APTSSSTKKT QLQLEHFLLD FQMILNGINN |
| Sequenz 3: | APTSSSTKKT QLQLEHFLMK FQMILNGINN |

hergestellt und getestet.

### Zytotoxizitätstest:

Zur Zytotoxizitätsmessung wurden NK-Zellen, die spezifisch Tumorzellen abtöten, als Effektorzellen mit Tumorzellen als Targetzellen in Gegenwart der Testpeptide inkubiert. Die Messung der Zytotoxizität erfolgte durch Messung der Lactatdehydrogenase (LDH)-Freisetzung nach Standard protokoll mit einem Zytotoxizitätstestkit (Promega, Deutschland) und einem Mikrotiterplattenreader. Als NK-Zellen (E) wurden YT-Zellen (DSMZ, Deutschland) eingesetzt. Als Targetzellen (T) wurden Daudi Zellen (DSMZ, Deutschland) verwendet. YT und Daudi Zellen wurden in Mikrotiterplatten im Verhältnis 1:10 ausgesät und in Gegenwart von 0.3, 3, 30 µM Testpeptid für 16 h inkubiert. Als Positivkontrolle diente humanes IL-2 (Sigma) in einer Konzentration von 5 nM.

Am nächsten Tag wurden je 10 µl Lysispuffer zu den Zellen gegeben und die Zellen wurden für weitere 2 h inkubiert. 5 µl Medium wurden anschließend in eine zweite Mikrotiterplatte überführt und mit 50 µl Substratpuffer versetzt Nach 30 min Inkubation wurde die Reaktion mit 50 µl Stopplösung beendet und die Absorption des entstandenen MTT-Farbkomplexes mittels einer Mikrotiterplattenreaders gemessen.

Wie in Tabelle 1 gezeigt, zeigten die beschriebenen Testpeptide bereits in einer Konzentration von 0,3 µM einen gleich großen Zytotoxizität-induzierenden Effekt wie humanes IL-2.

**Tabelle 1: Ergebnisübersicht des Zytotoxizitätstest**

| Substanz (Sequenz #) | Zytotoxizität |
|---|---|
| Kontrolle | 10% |
| IL-2 | 60% |
| Sequenz 1 30µM | 30% |
| Sequenz 1 3µM | 30% |
| Sequenz 1 0,3µM | 30% |
| Sequenz 2 30 µM | 10% |
| Sequenz 2 3µM | 80% |
| Sequenz 2 0,3µM | 30% |
| Sequenz 3 30µM | 0% |
| Sequenz 3 3µM | 20% |
| Sequenz3 0,3µM | 0% |

## Patentansprüche

1. Bindungsmolekül, bestehend aus einer Trägerstruktur aus mindestens einer zyklischen Moleküluntereinheit und mindestens zwei Seitenkettenuntereinheiten, wobei die Seitenkettenuntereinheiten Polypeptidketten sind, die aus natürlichen und/oder nicht natürlichen D- und/oder L-Aminosäuren aufgebaut sind, und/oder Polynucleotidketten sind, und die Seitenkettenuntereinheiten mit der Trägerstruktur über Abstandhaltermoleküleuntereinheiten (Spacer) kovalent verbunden sind, **dadurch gekennzeichnet, dass** das Bindungsmolekül die Benzoltriamidstruktur 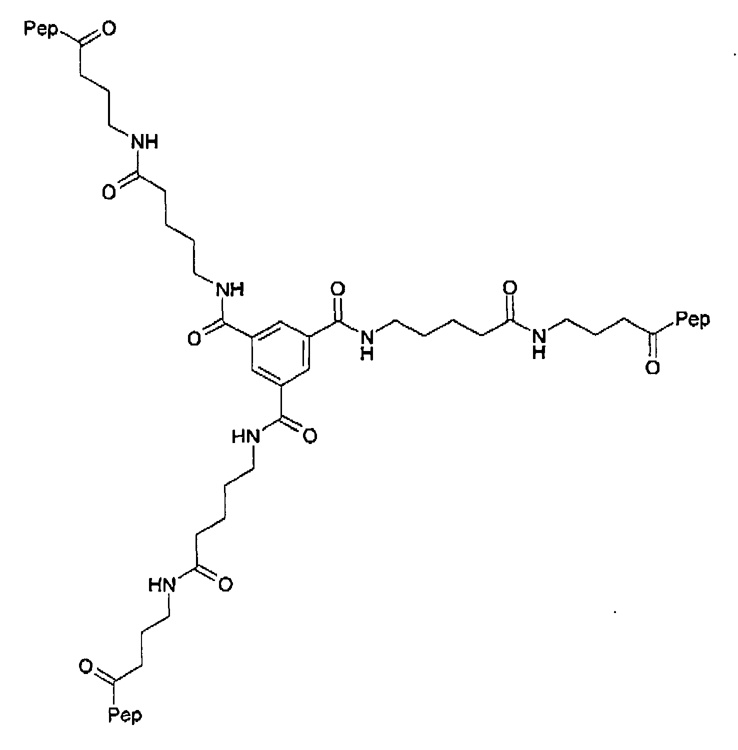 aufweist, wobei Pep für die Seitenkettenuntereinheiten steht.

2. Bindungsmolekül nach Anspruch 1, **dadurch gekennzeichnet, dass** die Polypeptidketten die gleiche Aminosäuresequenz aufweisen,

3. Bindungsmolekül nach Anspruch 1 und/oder 2, **dadurch gekennzeichnet, dass** die Polypeptidketten aus 5 bis 60, vorzugsweise 10 bis 50, insbesondere 12 bis 40 Aminosäuren bestehen.

4. Bindungsmolekül nach mindestens einem der Ansprüche 1 bis 3, **dadurch gekennzeichnet, dass** die nicht natürlichen Aminosäuren solche der Formel NH₂(CH₂)ₙCOOH mit n = 2 bis 8, insbesondere NH₂(CH₂)₂COOH, NH₂(CH₂)₃COOH oder NH₂(CH₂)₄COOH sind.

5. Bindungsmolekül nach mindestens einem der Ansprüche 1 bis 4, wobei die Seitenkettenuntereinheiten mindestens eine der Sequenzen
• APTSSSTKKT₁ QLQLEHX¹X²X³X⁴ X⁵QMILNGINN oder
• TIVX⁶FLNRWIT FX⁷QSX⁸ISTLT₁ aufweisen,
wobei
X¹ ausgewählt ist aus I oder L,
X² ausgewählt ist aus I oder L,
X³ ausgewählt ist aus V, L oder M,
X⁴ ausgewählt ist aus E, D oder K,
X⁵ ausgewählt ist aus L oder F,
X⁶ ausgewählt ist aus E oder D,
X⁷ ausgewählt ist aus A, G oder C,
X⁸ ausgewählt ist aus A oder I; und
an den Threonin T₁ zum Schutz vor proteolytischem Abbau eine Schutzgruppe nach dem Stand der Technik, bevorzugt ein Kohlenhydratrest, bevorzugt ausgewählt aus Glucose, Mannose, N-Acetylglucosamin oder N-Acetylgalactosamin auf geeignete Weise kovalent gebunden sein kann; sowie Bindungsmoleküle, bei denen die Seitenkettenuntereinheiten zu den angegebenen Sequenzen mindestens 80% Sequenzhomologie aufweisen.

6. Bindungsmolekül nach Anspruch 5 mit dem Aufbau
• APTSSSTKKT₁ QLQLEHX¹X²X³X⁴ X*⁵*QMILNGINN *-Trägerstruktur-*TIVX⁶FLNRWIT FX⁷QSX⁸ISTLT₁;
• TIVX⁶FLNRWIT FX⁷QSX⁸ISTLT₁ *-Trägerstruktur-* APTSSSTKKT₁ QLQLEHX¹X²X³X⁴ X⁵QMILNGINN;
• APTSSSTKKT₁ QLQLEHX¹X²X³X⁴ X⁵QMILNGINN *-Trägerstruktur-*APTSSSTKKT₁ QLQLEHX⁰¹X⁰²X⁰³X⁰⁴ X⁰⁵QMILNGINN oder
• TIVX⁶FLNRWIT FX⁷QSX⁸ISTLT₁ *-Trägerstruktur-* TIVX⁰⁶FLNRWIT FX⁰⁷QSX⁰⁸ISTLT₁
wobei die Aminosäuren X⁰¹ ,X⁰² ,X⁰³ , X⁰⁴ und X⁰⁵ ausgewählt werden wie für X¹, X², X³ ,X⁴ und X⁵ angegeben.

7. Bindungsmolekül nach mindestens einem der Ansprüche 1 bis 6 , wobei mindestens eine Seitenkettenuntereinheit die Sequenz
SKNEKALGRKIX₁X₂X₃X₄SSRX₅GHSFLS, mit
X₁= N oder L
X₂= S oder Q oder L oder E
X₃= W oder L oder R oder A oder Y
X₄ = E oder N oder A oder D oder H
X₅= S oder A aufweist,
sowie Bindungsmoleküle, bei denen die Seitenkettenuntereinheiten zu den angegebenen Sequenzen mindestens 80% Sequenzhomologie aufweisen.

8. Bindungsmolekül nach Anspruch 1, **dadurch gekennzeichnet, dass** es eine der Strukturformeln 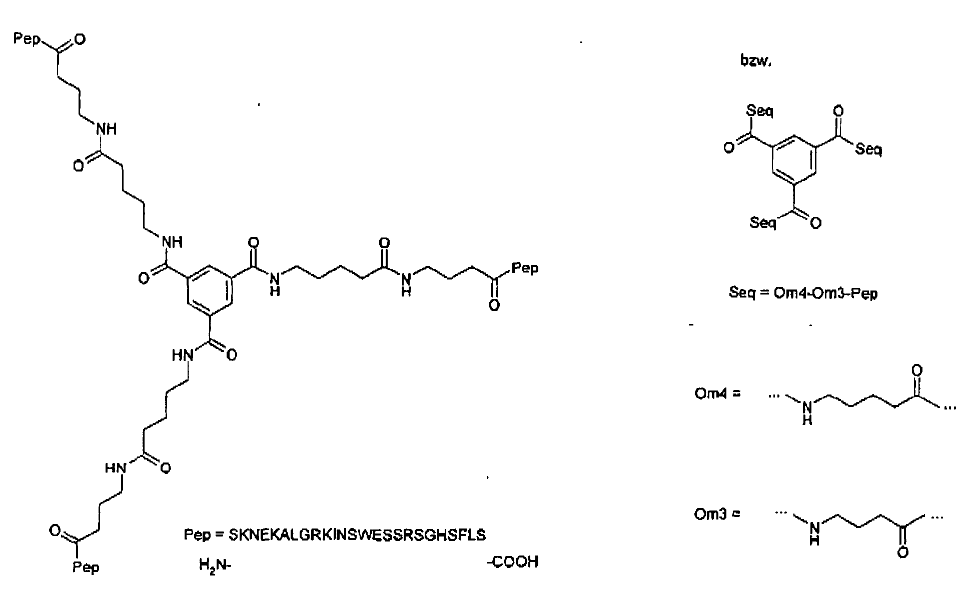 aufweist, sowie Bindungsmoleküle, bei denen die Seitenkettenuntereinheiten zu den angegebenen Sequenzen mindestens 80% Sequenzhomologie aufweisen.

9. Verfahren zur Herstellung eines Bindungsmoleküls nach mindestens einem der Ansprüche 1 bis 8, bei dem das Verfahren als Festphasensyntheseverfahren durchgeführt wird, bei der das Peptid der Seitenkettenuntereinheit an der Festphase gebunden sukzessive mit den entsprechenden Aminosäuren verlängert wird, wobei sich gegebenenfalls im letzten Schritt eine Ankopplung an die Trägerstruktur anschließt.

10. Arzneimittel, enthaltend Bindungsmoleküle nach einem der Ansprüche 1 bis 9.

11. Diagnostikmittel, enthaltend Bindungsmoleküle nach einem der Ansprüche 1 bis 9.

12. Verwendung von Bindungsmolekülen nach einem der Ansprüche 1 bis 9 zur Herstellung eines Arzneimittels oder Diagnostikmittels zur Behandlung oder zum Nachweis von Erkrankungen des Immunsystems, insbesondere Entzündungen, arthritische Prozesse, Immundefizienzsyndrome, Autoimmunsyndrome und Immunschwächesyndrome, Fertilitätsstörungen, zur Kontrazeption oder antiviralen Prophylaxe, Erkrankungen, die mit einer erhöhten Zellproliferation in Zusammenhang stehen, insbesondere Tumorerkrankungen, Krebserkrankungen, Carcinomen, Sarkomen, Lymphomen und Leukämien; und/oder infektiösen Prozessen beim Menschen.

13. Arzneimittel nach Anspruch 10 in Form einer Mikroverkapselung, liposomalen Zubereitung oder Depotpräparation, enthaltend geeignete Zusatz-, Träger-, Hilfsstoffe und/oder mindestens einen weiteren Wirkstoff.

14. Arzneimittel nach Anspruch 10, **dadurch gekennzeichnet, dass** als weiterer Wirkstoff Interleukin 12, ein Interleukin 12 Rezeptor spezifisches Bindungs- und Effektormolekül oder rekombinantes IL12 verwendet wird.

15. Arzneimittel gemäß Anspruch 10, **dadurch gekennzeichnet, dass** als weiterer Wirkstoff TRAIL (TNF-related apoptosis inducing ligand), ein TRAIL-Rezeptor spezifisches Bindungs- und Effektormolekül oder rekombinant hergestelltes TRAIL verwendet wird.

16. Arzneimittel gemäß Anspruch 10, **dadurch gekennzeichnet, dass** als weiterer Wirkstoff mindestens ein Virostatikum verwendet wird, insbesondere ein Virostatikum, das imstande ist, den Eintritt von Viruspartikeln, insbesondere HIV-Partikeln, in Zielzellen, insbesondere T-Lymphozyten, zu verlangsamen oder zu blockieren.

## Claims

1. A binding molecule consisting of a support structure of at least one cyclic molecular subunit and at least two side chain subunits, wherein said side chain subunits are polypeptide chains consisting of natural and/or unnatural D- and/or L-amino acids, and/or polynucleotide chains, and the side chain subunits are covalently bonded to the support structure by spacer molecular subunits (spacers), **characterized in that** said binding molecule has the benzenetriamide structure: 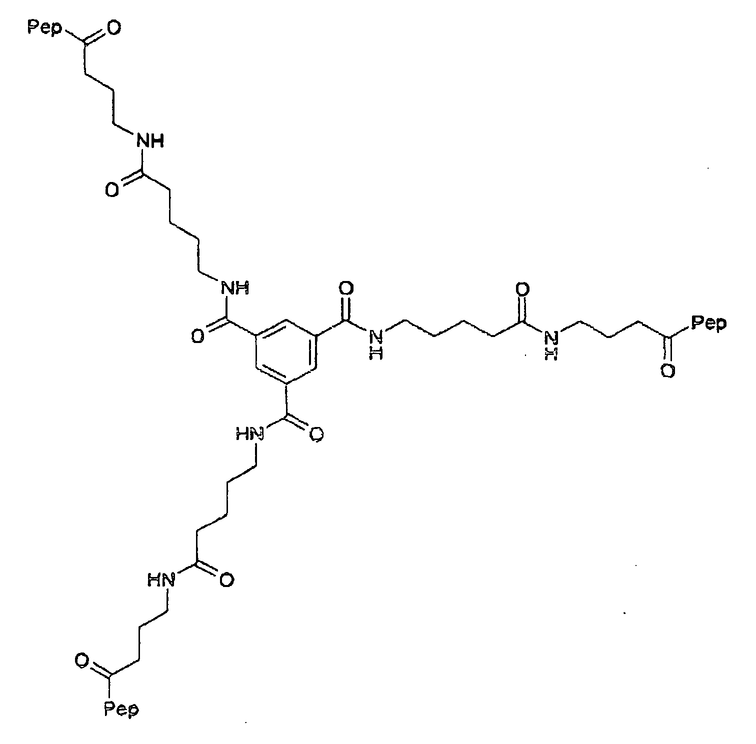 wherein Pep represents the side chain subunits.

2. The binding molecule of claim 1, **characterized in that** the polypeptide chains have the same amino acid sequence.

3. The binding molecule of claim 1 and/or 2, **characterized in that** the polypeptide chains consist of 5 to 60, preferably 10 to 50, in particular 12 to 40 amino acids.

4. The binding molecule of at least one of claims 1 to 3, **characterized in that** the unnatural amino acids have the formula NH₂(CH₂)ₙCOOH with n = 2 to 8, in particular NH₂(CH₂)₂COOH, NH₂(CH₂)₃COOH or NH₂(CH₂)₄COOH.

5. The binding molecule of at least one of claims 1 to 4, wherein the side chain subunits have at least one of the sequences
APTSSSTKKT₁ QLQLEHX¹X²X³X⁴ X⁵QMILNGINN or
TIVX⁶FLNRWIT FX⁷QSX⁸ISTLT₁
wherein
X¹ is selected from I or L,
X² is selected from I or L,
X³ is selected from V, L or M,
X⁴ is selected from E, D or K,
X⁵ is selected from L or F,
X⁶ is selected from E or D,
X⁷ is selected from A, G or C,
X⁸ is selected from A or I and
at the T₁ threonine a protective group according to the state of the art, preferably a carbohydrate moiety, preferably selected from glucose, mannose, N-acetylglucosamine or N-acetylgalactosamine, may be covalently bonded in a suitable manner as a protection against proteolytic degradation;
and binding molecules, wherein the side chain subunits have sequence homologies to the stated sequences of at least 80 %.

6. The binding molecule of claim 5 having the structure
APTSSSTKKT₁ QLQLEHX¹X²X³X⁴ X⁵QMILNGINN - *support structure -* TIVX⁶FLNRWIT FX⁷QSX⁸ISTLT₁;
TIVX⁶FLNRWIT FX⁷QSX⁸ISTLT₁ - *support structure -* APTSSSTKKT₁ QLQLEHX¹X²X³X⁴ X⁵QMILNGINN;
APTSSSTKKT₁ QLQLEHX¹X²X³X⁴ X⁵QMILNGINN - *support structure -* APTSSSTKKT₁ QLQLEHX⁰¹X⁰²X⁰³X⁰⁴ X⁰⁵QMILNGINN or
TIVX⁶FLNRWIT FX⁷QSX⁸ISTLT₁ - support structure - TIVX⁰⁶FLNRWIT FX⁰⁷QSX⁰⁸ISTLT₁,
wherein the amino acids X⁰¹, X⁰², X⁰³, X⁰⁴ and X⁰⁵ are selected as indicated for X¹, X², X³, X⁴ and X⁵.

7. The binding molecule of at least one of claims 1 to 6, wherein at least one side chain subunit has the sequence
SKNEKALGRKIX₁X₂X₃X₄SSRX₅GHSFLS, with
X₁ = N or L,
X₂ = S or Q or L or E,
X₃ = W or L or R or A or Y,
X₄ = E or N or A or D or H,
X₅ = S or A,
and binding molecules wherein the side chain subunits have sequence homologies to the stated sequences of at least 80 %.

8. The binding molecule of claim 1, **characterized in that** it has one of the structural formulas 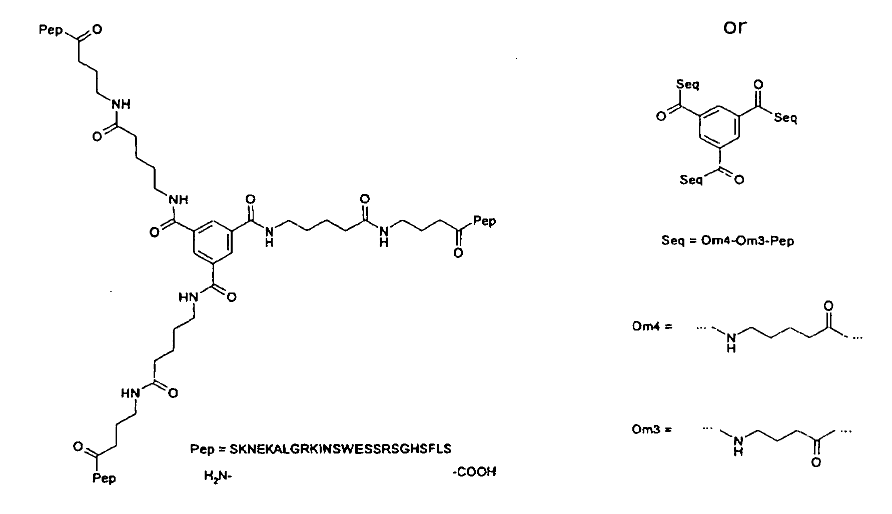 and binding molecules wherein the side chain subunits have sequence homologies to the stated sequences of at least 80 %.

9. A process for the preparation of a binding molecule according to at least one of claims 1 to 8, wherein the process is performed as a solid phase synthesis process wherein the peptide of the side chain subunit is bonded to the solid phase, successively extended by the respective amino acids and optionally a coupling to the support structure follows in a last step.

10. A pharmaceutical containing binding molecules according to any of claims 1 to 9.

11. A diagnostic agent containing binding molecules according to any of claims 1 to 9.

12. Use of binding molecules according to any of claims 1 to 9 for preparing a pharmaceutical or diagnostic agent for the treatment or detection of diseases of the immune system, in particular inflammations, arthritic processes, immunodeficiency syndromes, auto-immune syndromes and immunodeficiency syndromes, fertility disturbances, for contraception or antiviral prophylaxis, diseases connected with increased proliferation of cells, in particular tumor diseases, cancers, carcinomas, sarcomas, lymphomas and leukemias; and/or infectious processes in humans.

13. The pharmaceutical according to claim 10 in the form of a microencapsulation, liposomal preparation or depot preparation containing suitable additives, carriers, adjuvants and/or at least one additional active substance.

14. The pharmaceutical according to claim 10, **characterized in that** interleukin 12, an interleukin 12 receptor-specific binding and effector molecule or recombinant IL12 is used as the additional active substance.

15. The pharmaceutical according to claim 10, **characterized in that** TRAIL (TNF related apoptosis inducing ligand), a TRAIL receptor specific binding and effector molecule or recombinantly prepared TRAIL is used as the additional active substance.

16. The pharmaceutical according to claim 10, **characterized in that** a virostatic, in particular a virostatic capable of retarding or blocking the entry of virus particles, in particular HIV particles, into target cells, in particular T lymphocytes, is used as the additional active substance.

## Revendications

1. Molecule de liaison, composee d'une structure-support comptant au moins une sous-unite de molecule cyclique et au moins deux sous-unites de chaîne laterale, dans laquelle les sous-unites de chaîne laterale sont des chaînes polypeptidiques, qui sont constituees de D- et/ou L-acides amines naturels et/ou non naturels, et/ou de chaînes polynucleotidiques, et les sous-unites de chaîne laterale sont liees par une liaison covalente à la structure-support par l'intermediaire de sous-unites de molecules d'espacement (espaceurs), **caracterisee en ce que** la molecule de liaison presente la structure benzènetriamide 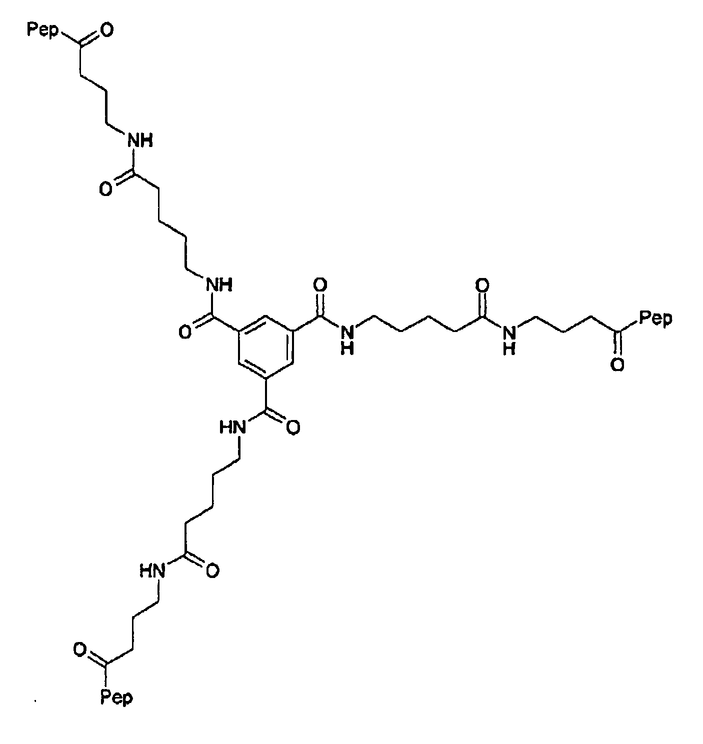 dans laquelle Pep represente les sous-unites de chaîne laterale.

2. Molecule de liaison selon la revendication 1, **caracterisee en ce que** les chaînes polypeptidiques presentent la mdme sequence d'acides amines.

3. Molecule de liaison selon la revendication 1 et/ou 2, **caracterisee en ce que** les chaînes polypeptidiques comptent 5 à 60, de preference 10 à 50, en particulier 12 à 40 acides amines.

4. Molecule de liaison selon au moins l'une quelconque des revendications 1 à 3, **caracterisee en ce que** les acides amines non naturels sont ceux de formule NH₂(CH₂)ₙCOOH où n = 2 à 8, en particulier NH₂(CH₂)₂COOH, NH₂(CH₂)₃COOH ou NH₂(CH₂)₄COOH.

5. Molecule de liaison selon au moins l'une quelconque des revendications 1 à 4, dans laquelle les sous-unites de chaîne laterale presentent au moins l'une des sequences
• APTSSSTKKT₁ QLQLEHX¹X²X³X⁴ X⁵QMILNGINN ou
• TIVX⁶FLNRWIT FX⁷QSX⁸ISTLT₁,
où
X¹ est choisi parmi I ou L,
X² est choisi parmi I ou L,
X³ est choisi parmi V, L ou M,
X⁴ est choisi parmi E, D ou K,
X⁵ est choisi parmi L ou F,
X⁶ est choisi parmi E ou D,
X⁷ est choisi parmi A, G ou C,
X⁸ est choisi parmi A ou I ; et
un groupe protecteur selon l'etat de la technique, de preference un groupe glucidique, choisi de preference parmi le glucose, le mannose, la N-acetylglucosamine ou la N-acetylgalactosamine, pouvant dtre lie de manière appropriee par une liaison covalente à la threonine T₁ pour la protection contre une degradation proteolytique ; et molecules de liaison, dans lesquelles les sous-unites de chaîne laterale presentent une homologie de sequence d'au moins 80% avec les sequences indiquees.

6. Molecule de liaison selon la revendication 5 de structure
• APTSSSTKKT₁ QLQLEHX¹X²X³X⁴ X⁵QMILNGINN - *structure-support-* TIVX⁶FLNRWIT FX⁷QSX⁸ISTLT₁ ;
• TIVX⁶FLNRWIT FX⁷QSX⁸ISTLT₁ -structure-*support-* APTSSSTKKT₁ QLQLEHX¹X²X³X⁴ X⁵QMILNGINN ;
• APTSSSTKKT₁ QLQLEHX¹X²X³X⁴ X⁵QMILNGINN -structure-*support-* APTSSSTKKT₁ QLQLEHX⁰¹X⁰²X⁰³X⁰⁴ X⁰⁵QMILNGINN ou
• TIVX⁶FLNRWIT FX⁷QSX⁸ISTLT₁ -structure-*support-* TIVX⁰⁶FLNRWIT FX⁰⁷QSX⁰⁸ISTLT₁
dans laquelle les acides amines X⁰¹, X⁰², X⁰³, X⁰⁴ et X⁰⁵ sont choisis de la mdme façon que X¹, X², X³, X⁴ et X⁵.

7. Molecule de liaison selon au moins l'une quelconque des revendications 1 à 6, dans laquelle au moins une sous-unite de chaîne laterale presente la sequence
SKNEKALGRKIX₁X₂X₃X₄SSRX₅GHSFLS, où
X₁ = N ou L
X₂ = S ou Q ou L ou E
X₃ = W ou L ou R ou A ou Y
X₄ = E ou N ou A ou D ou H
X₅ = S ou A,
et molecules de liaison, dans lesquelles les sous-unites de chaîne laterale presentent une homologie de sequence d'au moins 80% avec les sequences indiquees.

8. Molecule de liaison selon la revendication 1, **caracterisee en ce qu'**elle presente l'une des formules developpees 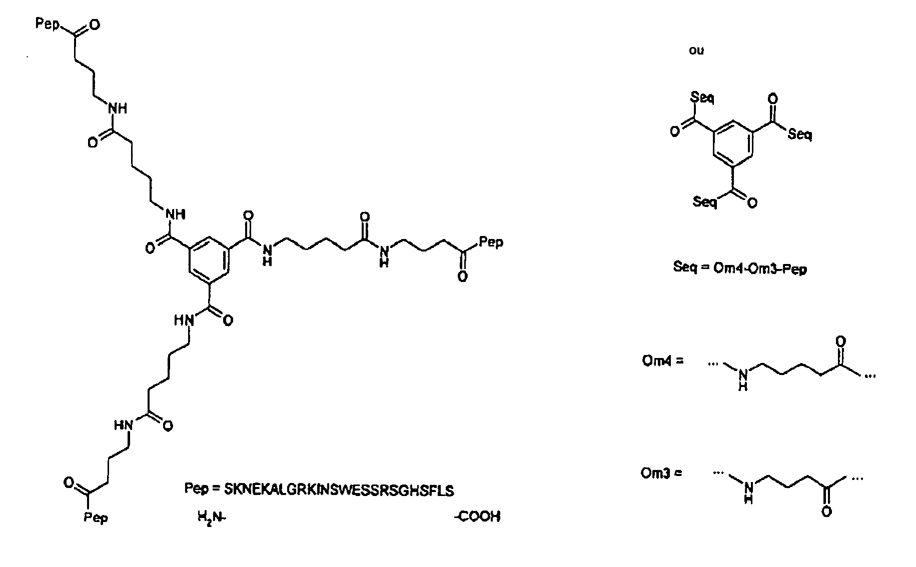 et molecules de liaison, dans lesquelles les sous-unites de chaîne laterale presentent une homologie de sequence d'au moins 80% avec les sequences indiquees.

9. Procede de production d'une molecule de liaison selon au moins l'une quelconque des revendications 1 à 8, dans lequel le procede est realise en tant que procede de synthèse en phase solide, dans lequel le peptide de la sous-unite de chaîne laterale lie à la phase solide est successivement rallonge avec les acides amines correspondants, dans lequel un couplage à la structure-support est eventuellement realise à la dernière etape.

10. Medicament contenant des molecules de liaison selon l'une quelconque des revendications 1 à 9.

11. Agent diagnostique contenant des molecules de liaison selon l'une quelconque des revendications 1 à 9.

12. Utilisation de molecules de liaison selon l'une quelconque des revendications 1 à 9 pour la fabrication d'un medicament ou d'un agent diagnostique destine au traitement ou au depistage de maladies du système immunitaire, en particulier d'inflammations, de processus arthritiques, de syndromes d'immunodeficience, de syndromes autoimmuns et de syndromes de deficience immunitaire, de problèmes de fertilite, pour la contraception ou la prophylaxie antivirale, les maladies liees à proliferation cellulaire accrue, en particulier les maladies tumorales, les maladies cancereuses, les carcinomes, les sarcomes, les lymphomes et les leucemies ; et/ou des processus infectieux chez l'dtre humain.

13. Medicament selon la revendication 10 sous la forme d'une micro-encapsulation, d'une preparation liposomale ou d'une preparation retard, contenant des excipients, vehicules, adjuvants appropries et/ou au moins un autre principe actif.

14. Medicament selon la revendication 10, **caracterise en ce que** l'on utilise, en tant qu'autre principe actif, l'interleukine 12, une molecule de liaison ou effectrice specifique du recepteur de l'interleukine 12, ou IL12 recombinee.

15. Medicament selon la revendication 10, **caracterise en ce que** l'on utilise, en tant qu'autre principe actif, TRAIL (TNF-related apoptosis inducing ligand), une molecule de liaison ou effectrice specifique du recepteur de TRAIL, ou TRAIL obtenu par recombinaison.

16. Medicament selon la revendication 10, **caracterise en ce que** l'on utilise, en tant qu'autre principe actif, au moins un virostatique, en particulier un virostatique qui est capable de ralentir ou de bloquer la penetration de particules virales, en particulier de particules du VIH, dans les cellules cibles, en particulier les lymphocytes T.
